# EUROPEAN PATENT SPECIFICATION

(11) **EP 3 732 403 B1**
(45) Date of publication and mention of the grant of the patent: **12.07.2023**
(21) Application number: 18833679.6
(22) Date of filing: 27.12.2018
(51) Int. Cl.: F24F 7/00, F24F 13/02, F24F 11/52, F24F 11/89, F24F 110/10, F24F 110/20, F24F 110/50, F24F 110/72, F24F 110/65

(54) **SENSING DEVICE AND METHOD OF SENSING PROPERTIES OF AIR**
SENSORVORRICHTUNG UND VERFAHREN ZUR MESSUNG VON LUFTEIGENSCHAFTEN
DISPOSITIF DE DÉTECTION ET PROCÉDÉ DE DÉTECTION DE PROPRIÉTÉS D'AIR

(30) Priority: 28.12.2017 FI 20176181
(43) Date of publication of application: 04.11.2020
(73) Proprietor: Loopshore OY, 33200 Tampere (FI)
(72) Inventor: EDGREN, Janne, 33400 Tampere (FI)
(74) Representative: Papula Oy
(86) International application number: PCT/FI2018/050981
(87) International publication number: WO 2019/129932

(56) References cited:
- EP-A1- 3 165 839
- EP-A1- 3 211 338
- WO-A1-2007/031932
- GB-A- 2 539 449

## Description

The invention relates to a sensing device which is intended to determine properties of air or properties of airborne particles. The sensing device comprises at least

The invention further relates to a method of determining properties of air or properties of airborne particles.

The field of the invention is defined more specifically in the preambles of the independent claims.

Indoor air quality (IAQ) is an important consideration in creating a satisfactory environment in office buildings, schools, and health care facilities. Indoor air contaminants can originate within the building or be drawn in from outdoors. Known air quality sensing devices are clumsy and expensive laboratory equipment or stationary wall or ceiling mounted complex systems.

### Brief description of the invention

An object of the invention is to provide a novel and improved sensing device. The invention further relates to a method of sensing properties of air.

The sensing device according to the invention is characterized by the characterizing features of the independent apparatus claim 1.

The method according to the invention is characterized by the characterizing features and steps of the independent method claim 13.

An idea of the disclosed solution is that indoor air quality (IAQ) may be determined by means of a portable sensing device which comprises one or more sensing units for determining properties of surrounding air by means of one or more air sensors. The device further comprises one or more indicating units for indicating the detected air quality. The sensing device is provided with a body, which comprises at least one elongated air channel element. Both ends of the air channel element are open, whereby air flow may be conveyed through it. The mentioned sensing unit is mounted inside the air channel element, whereby the air sensors of the unit are configured to sense properties of the air flow. Further, the mentioned indicating device comprises at least one light source for providing visual indication of the detected air quality. In other words, air quality inside the air channel element is detected and the results are indicated visually by illuminating the air channel element with different colors or effects in response to the determined air quality.

An advantage of the disclosed solution is that the visual indication of the air quality is easy to see by all persons occupying the monitored indoor space. The device has elegant appearance since the air channel element is illuminated instead of using conventional indicating lamps providing spot-like light beams. Since the sensing device is portable it is easy to move it to different rooms and indoor spaces, and to place it to desired locations in these spaces. Further, the portable device may easily be taken along so that the device may monitor the surrounding air where ever the person visits during the day.

A further advantage is that, the sensing unit is mounted inside the air channel element wherein it is well protected against external forces and conditions. This way, the sensing unit and the air sensor are not damaged during transport and when the sensing device is positioned in harsh circumstances. An additional advantage is that the sensing device may have esthetically pleasant appearance and the device may have desired design when technical components are arranged inside the structure and are not visible to outside.

Preferred embodiments of the invention are provided in the dependent claims.

### Brief description of the figures

Some embodiments of the invention are described in more detail in the accompanying drawings, in which
Figure 1 is a schematic view of a sensing device for determining and monitoring air quality,
Figure 2 is a schematic top view of a sensing device fastened to an observation point,
Figure 3 is a schematic view of a mounting element of a sensing device,
Figure 4 is a schematic top view of a sensing device provided with an air channel element with rectangular shape,
Figures 5 - 8 are schematic and partly cross sectional views showing some alternative ways of illuminating an air channel element of a sensing device with light sources,
Figure 9 is a diagram showing that the sensing device may be provided with various sensors for detecting features relating to healthy air and environment, and
Figure 10 is a schematic top view of the solution of Figure 1 and illustrates location of the light source.

For the sake of clarity, the figures show some embodiments of the disclosed solution in a simplified manner. In the figures, like reference numerals identify like elements.

### Detailed description of some embodiments

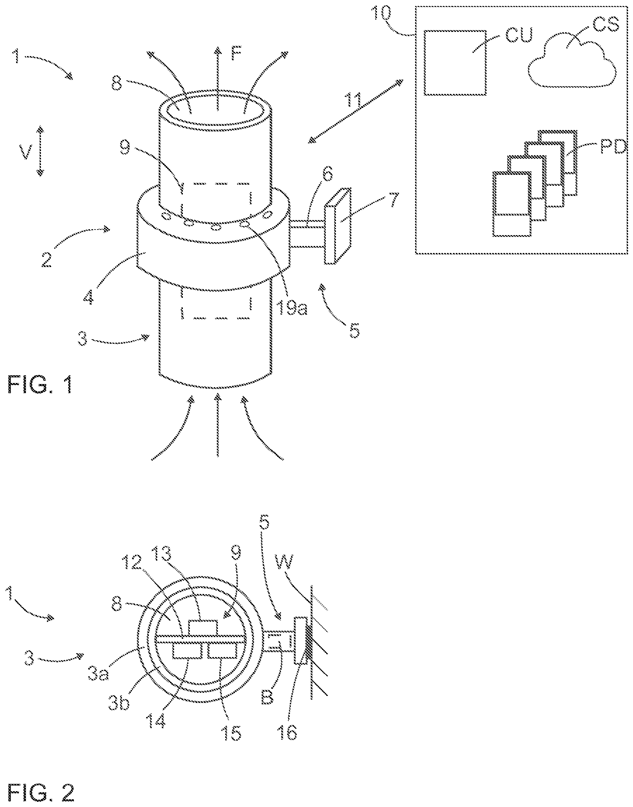

Figure 1 shows a sensing device 1 comprising a body 2, which is provided with an elongated air channel element 3 having vertical V orientation. The body 2 has a base body 4, which may have a ring-shaped configuration. The base body supports the elongated air channel element 3 and is connected to a mounting element 5 by means of which the sensing device 1 may be fastened to a desired observation position. The mounting element 5 may comprise a shaft 6 and connecting means 7, such as mechanical or magnetic fastening means. The mounting element 5 allows the sensing device to be mounted in a removable manner.

The air channel element 3 has open ends and natural air flow F may flow through a flow channel 8 or air detection channel, as is indicated by means of arrows. Inside the flow channel 8 is a sensing unit 9 comprising one or more air sensors. The sensing unit 9 may communicate with one or more external devices 10 by means of a data communication connection 11. Thus, the sensing device 1 may send and receive data to and from external servers, control units CU, cloud services CS, computers and portable electronic devices PD, such as smart phones, for example. The sensing device 9 may transmit gathered raw data to one or more external devices 10 for being analysed and may receive control commands for executing visualization of current state of air quality.

Figure 2 discloses a sensing device 1 wherein an air channel element 3 is a tubular piece comprising an outer tube 3a and an inner tube 3b inside the outer tube 3a. Inside a flow channel 8 is a sensing unit 9, which may comprise a circuit card 12 opposite edges of which are supported directly against inner surfaces of the inner tube 3b. One or more air sensors 13 may be mounted on the circuit card 12 as well as a data transmission unit 14. Further, one or more control units 15 may also be arranged on the circuit card 12 when needed. The sensing device 1 may be mounted to a wall surface W or corresponding mounting place by means of a mounting element 5 comprising a magnetic fastener 16. Then the sensing device 1 is very easy to mount and detach. The sensing device 1 may comprise an energy storage, such as battery B for providing the sensing unit 9 with needed electrical energy. The battery B may be located in connection with the mounting element 5.

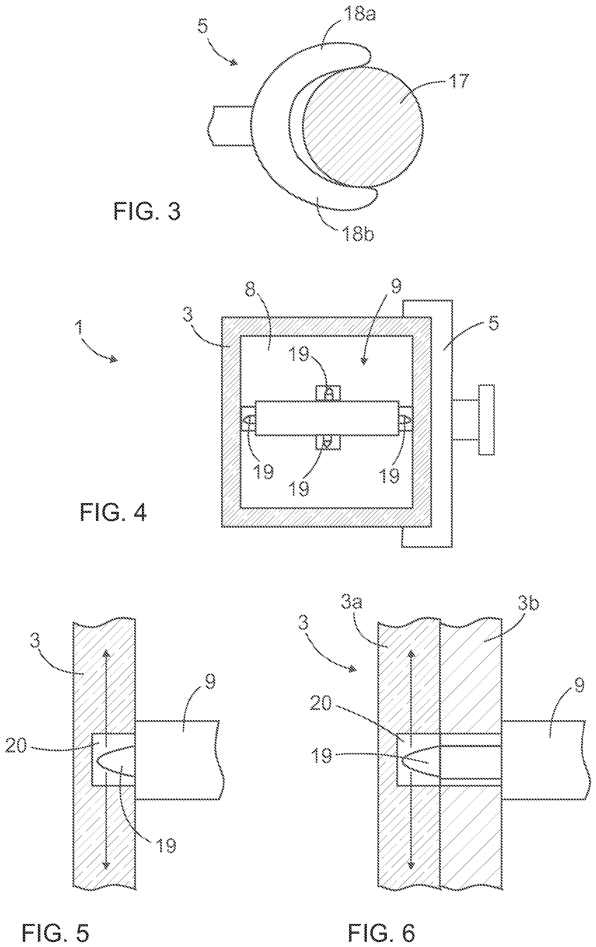

Figure 3 discloses an alternative mounting element 5, which is intended to be mounted to a bar 17 or corresponding element. The mounting element 5 may comprise clamp arms 18a, 18b which are pressed against the bar 17.

Figure 4 discloses a sensing device 1 comprising an air channel element 3 the shape of which is rectangular. Inside the air channel element 3 is a flow channel 8 provided with a sensing unit 9. The sensing unit comprises one or more air sensors and other needed components even though they are not shown in Figure 4. However, Figure 4 discloses that the sensing unit 9 may comprise one more light sources 19 for illuminating the air channel element 3, which may be made of transparent material.

In Figure 5 an air channel element 3 comprises a recess 20 at a sensing unit 9. A light source 19, such as a led bulb, is arranged inside the recess 20 so that light beam may be conveyed inside material of the element 3.

In Figure 6 the air channel element 3 comprises an outer tube 3a or element and an inner tube 3b or element. The inner tube 3b may be made of opaque material. A recess 20 is formed in order to allow a light source to be mounted inside the transparent outer tube 3a.

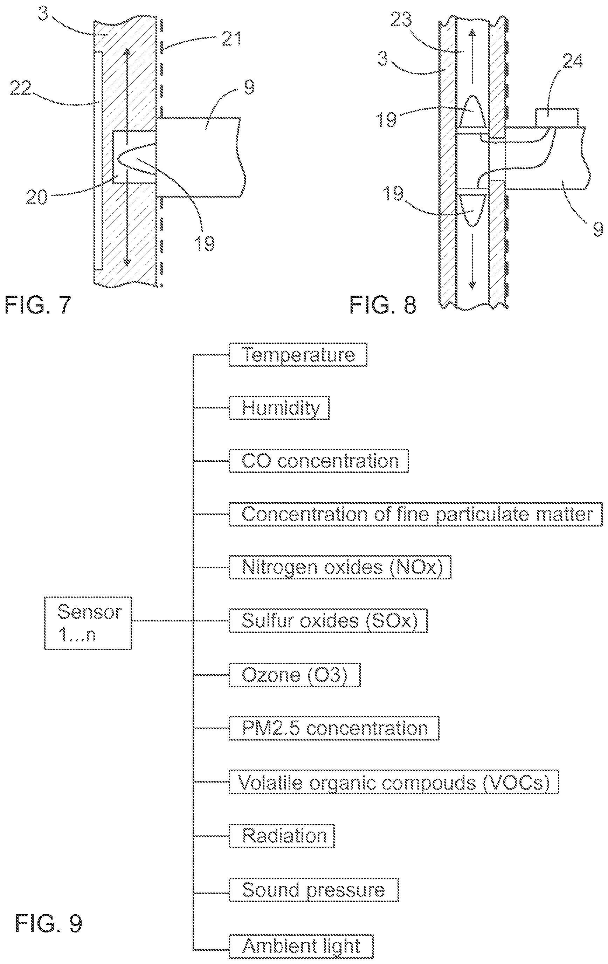

Figure 7 discloses an embodiment wherein an inner surface of the air channel element 3 is provided with an opaque coating 21 or surface treating in order to prevent inserted components to be seen from outside. Further, on outer surfaces of the element 3 may be one or more grooves 22 or other surface shapes by means of which visual appearance of the illumination may be influenced.

In Figure 8 the air channel element 3 comprises a light channel 23 and the light sources 19 are mounted inside the channel 23. Light sources 19 may be controlled by means of a light controller 24, which may be located in the sensing unit 9.

Let it be further mentioned, that Figure 1 discloses that several light sources 19a, such as led bulbs, may be mounted to the ring-shaped body 4 so that they can illuminate the outer surface of the air channel element 3. The light sources 19a may thereby serve as root lights and they may serve as additional lights to those disclosed earlier in this patent application, or in some cases they may even substitute them.

Figure 9 discloses some features which may be monitored by means of the disclosed sensing device. The mentioned features are clear as such. However, the feature NOx is a generic term for the nitrogen oxides that are most relevant for air pollution, namely nitric oxide (NO) and nitrogen dioxide (NO2). NOx gases are usually produced from the reaction among nitrogen and oxygen during combustion of fuels in air; especially at high temperatures, such as occur in car engines. In large cities, the nitrogen oxides emitted can be a significant source of air pollution. Sulfur Dioxide (SO2) is one of a group of gases called sulfur oxides (SOx). While all of these gases are harmful to human health and the environment, SO2 is of greater concern. Volatile organic compounds (VOCs) are emitted as gases from certain solids or liquids. VOCs include a variety of chemicals, some of which may have short- and long-term adverse health effects.

Further, the device may be provided with an UV index sensor, ambient light sensor or any other dedicated light sensors, or with a combination light sensor. For example, by means of such light sensors it is possible to detect if lights are switched on in the monitored room space, which information is valuable for access control. The sensing data gathered by the light sensors may also be implemented for other monitoring purposes.

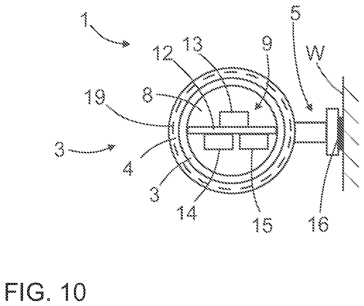

Figure 10 discloses that the light source 19 may be arranged at the mounting element 5 whereby direct beam of light is visible outside the device but instead the light source 19 illuminates the flow channel 8. The light source 19 may comprise several led bulbs which may be mounted to the mounting element 5 or to the flow channel 8.

The drawings and the related description are only intended to illustrate the idea of the invention. In its details, the invention may vary within the scope of the claims.

## Claims

1. A sensing device (1) for determining indoor air quality (IAQ), wherein the sensing device (1) comprises:
a body (2);
at least one sensing unit (9) for determining properties of surrounding air by means of at least one air sensor (13); and
at least one indicating unit for indicating the detected air quality;
and wherein the body (2) comprises at least one elongated air channel element (3) ends of which are open, thereby allowing air flow (F) through the air channel element (3);
the at least one sensing unit (9)is mounted inside the mentioned air channel element (3); and
the mentioned at least one indicating device comprises at least one light source (19) for providing visual indication of the detected air quality;
**characterized in that**
the mentioned at least one light source (19) is configured to illuminate at least an outer surface of the air channel element (3) in response to the detected indoor air quality, whereby the air channel element (3) is transparent and whereby the at least one light source (19) and the transparent air channel element (3) are configured to co-operate and to serve as a functional illuminating pair.

2. The sensing device as claimed in claim 1, **characterized in that**
the air channel element (3) is a tubular element.

3. The sensing device as claimed in claim 1 or 2, **characterized in that**
the air channel element (3) is at least partly transparent.

4. The sensing device as claimed in any one of the preceding claims 1 - 3, **characterized in that**
the sensing device (1) comprises several light sources (19) each of them locating at least partly inside structure of the air channel element (3) and configured to direct their light directly inside material of the air channel element (3).

5. The sensing device as claimed in any one of the preceding claims 1 - 4, **characterized in that**
color of the produced light of the light sources (19) is configured to change in response to the detected air quality.

6. The sensing device as claimed in any one of the preceding claims 1 - 5, **characterized in that**
the mentioned light sources (19) are led bulbs; and
color of the produced light of the led bulbs is configured to change in response to the detected air quality.

7. The sensing device as claimed in any one of the preceding claims 1 - 6, **characterized in that**
the body (2) comprises at least one mounting element (5) for mounting the sensing device (1) in a removable manner.

8. The sensing device as claimed in any one of the preceding claims 1 - 7, **characterized in that**
the sensing unit (9) is supported against inner surfaces of the air channel element (3).

9. The sensing device as claimed in any one of the preceding claims 1 - 8, **characterized in that**
the sensing device (1) has a vertical (V) mounting direction wherein the air channel element (3) is orientated vertically (V) and air is flowing vertically through the air channel.

10. The sensing device as claimed in any one of the preceding claims 1 - 9, **characterized in that**
the air channel element (3) comprises an opaque inner tube (3b) and a transparent outer tube (3a).

11. The sensing device as claimed in any one of the preceding claims 1 - 10, **characterized in that**
the sensing unit (19) comprises several air sensors; and
the air sensors (13) are configured to determine at least temperature, humidity, CO concentrations and concentrations of fine particulate matter.

12. The sensing device as claimed in any one of the preceding claims 1 - 11, **characterized in that**
the sensing unit (9) is connected to at least one control unit (CU, 15) which comprises at least one processor for processing data gathered by the at least one air sensor (13) and being transmitted to the control unit (CU, 15); and
the control unit (CU, 15) is configured to determine air quality properties and to control operation of the light sources (19) in response to the detected properties of the air.

13. A method of monitoring indoor air quality by means of a portable sensing device (1), the method comprises:
measuring properties of surrounding air by means of at least one sensing unit (19) comprising at least one air sensor (13);
determining the air quality in response to the gathered measuring data; and
indicating the determined air quality visually;
measuring the air quality inside an elongated air channel element (3) which envelopes the mentioned at least one sensing unit (19); and
illuminating at least partly the air channel element (3) with different colors in response to the determined air quality;
**characterized by**
illuminating at least an outer surface of the air channel element (3) in response to the detected indoor air quality by means of the mentioned at least one light source (19), whereby the at least one light source (19) and the transparent air channel element (3) co-operate and serve as a functional illuminating pair.

14. The method according to claim 13;
**characterized by**
measuring the air flowing naturally through the air channel element (3) which is orientated vertically (V).

## Patentansprüche

1. Sensorvorrichtung (1) zur Ermittlung der Innenraumluftqualität (IAQ), wobei die Sensorvorrichtung (1) umfasst:
einen Körper (2);
mindestens eine Erfassungseinheit (9) zur Ermittlung von Eigenschaften der Umgebungsluft durch mindestens einen Luftsensor (13); und
mindestens eine Anzeigeeinheit zur Anzeige der erfassten Luftqualität;
und wobei der Körper (2) mindestens ein längliches Luftkanalelement (3) umfasst, dessen Enden offen sind, wodurch ein Luftstrom (F) durch das Luftkanalelement (3) ermöglicht wird;
wobei die mindestens eine Erfassungseinheit (9) im Inneren des genannten Luftkanalelements (3) angebracht ist; und
die genannte mindestens eine Anzeigevorrichtung mindestens eine Lichtquelle (19) umfasst, um eine visuelle Anzeige der erfassten Luftqualität bereitzustellen;
**dadurch gekennzeichnet, dass**
die erwähnte mindestens eine Lichtquelle (19) so konfiguriert ist, dass sie mindestens eine Außenfläche des Luftkanalelements (3) in Reaktion auf die erfasste Innenraumluftqualität beleuchtet, wobei das Luftkanalelement (3) transparent ist und wobei die mindestens eine Lichtquelle (19) und das transparente Luftkanalelement (3) so konfiguriert sind, dass sie zusammenwirken und als ein funktionales Beleuchtungspaar dienen.

2. Sensorvorrichtung nach Anspruch 1, **dadurch gekennzeichnet, dass** das Luftkanalelement (3) ein rohrförmiges Element ist.

3. Sensorvorrichtung nach Anspruch 1 oder 2, **dadurch gekennzeichnet, dass** das Luftkanalelement (3) zumindest teilweise transparent ist.

4. Sensorvorrichtung nach irgendeinem der voranstehenden Ansprüche 1 - 3, **dadurch gekennzeichnet, dass** die Sensorvorrichtung (1) mehrere Lichtquellen (19) umfasst, von denen jede zumindest teilweise innerhalb der Struktur des Luftkanalelements (3) angeordnet und so konfiguriert ist, dass sie ihr Licht direkt in das Material des Luftkanalelements (3) richtet.

5. Sensorvorrichtung nach irgendeinem der voranstehenden Ansprüche 1 - 4, **dadurch gekennzeichnet, dass** die Farbe des von den Lichtquellen (19) erzeugten Lichts so konfiguriert ist, dass sie sich in Abhängigkeit von der erfassten Luftqualität ändert.

6. Sensorvorrichtung nach irgendeinem der voranstehenden Ansprüche 1 bis 5, **dadurch gekennzeichnet, dass** die genannten Lichtquellen (19) LED-Lampen sind; und die Farbe des von den LED-Lampen erzeugten Lichts so konfiguriert ist, dass sie sich in Abhängigkeit von der erfassten Luftqualität ändert.

7. Sensorvorrichtung nach irgendeinem der voranstehenden Ansprüche 1 - 6, **dadurch gekennzeichnet, dass** der Körper (2) mindestens ein Befestigungselement (5) zur lösbaren Befestigung der Sensorvorrichtung (1) aufweist.

8. Sensorvorrichtung nach irgendeinem der voranstehenden Ansprüche 1 - 7, **dadurch gekennzeichnet, dass** die Erfassungseinheit (9) gegen Innenflächen des Luftkanalelements (3) abgestützt ist.

9. Sensorvorrichtung nach irgendeinem der voranstehenden Ansprüche 1 - 8, **dadurch gekennzeichnet, dass** die Sensorvorrichtung (1) eine vertikale (V) Befestigungsrichtung aufweist, wobei das Luftkanalelement (3) vertikal (V) ausgerichtet ist und Luft vertikal durch den Luftkanal strömt.

10. Sensorvorrichtung nach irgendeinem der voranstehenden Ansprüche 1 - 9, **dadurch gekennzeichnet, dass** das Luftkanalelement (3) ein lichtundurchlässiges Innenrohr (3b) und ein transparentes Außenrohr (3a) aufweist.

11. Sensorvorrichtung nach irgendeinem der voranstehenden Ansprüche 1 - 10, **dadurch gekennzeichnet, dass** die Erfassungseinheit (19) mehrere Luftsensoren umfasst; und die Luftsensoren (13) so konfiguriert sind, dass sie mindestens die Temperatur, die Luftfeuchtigkeit, die CO-Konzentration und die Feinstaubkonzentration ermitteln.

12. Sensorvorrichtung nach irgendeinem der voranstehenden Ansprüche 1 - 11, **dadurch gekennzeichnet, dass** die Erfassungseinheit (9) mit mindestens einer Steuereinheit (CU, 15) verbunden ist, die mindestens einen Prozessor zur Verarbeitung der von dem mindestens einen Luftsensor (13) gesammelten und an die Steuereinheit (CU, 15) übertragenen Daten umfasst; und die Steuereinheit (CU, 15) so konfiguriert ist, dass sie die Eigenschaften der Luftqualität ermittelt und den Betrieb der Lichtquellen (19) in Abhängigkeit von den erfassten Eigenschaften der Luft steuert.

13. Verfahren zur Überwachung der Innenraumluftqualität mit Hilfe einer tragbaren Sensorvorrichtung (1), wobei das Verfahren umfasst:
Messen der Eigenschaften von Umgebungsluft mit Hilfe mindestens einer Erfassungseinheit (19), die mindestens einen Luftsensor (13) umfasst;
Ermitteln der Luftqualität in Abhängigkeit von den gesammelten Messdaten; und
visuelles Anzeigen der ermittelten Luftqualität;
Messen der Luftqualität im Inneren eines länglichen Luftkanalelements (3), das die genannte mindestens eine Erfassungseinheit (19) umhüllt; und
zumindest teilweises Beleuchten des Luftkanalelements (3) in unterschiedlichen Farben in Abhängigkeit von der ermittelten Luftqualität;
**gekennzeichnet durch**
Beleuchten mindestens einer Außenfläche des Luftkanalelements (3) in Reaktion auf die erfasste Innenraumluftqualität durch die genannte mindestens eine Lichtquelle (19), wobei die mindestens eine Lichtquelle (19) und das transparente Luftkanalelement (3) zusammenwirken und als funktionales Beleuchtungspaar dienen.

14. Verfahren nach Anspruch 13; **gekennzeichnet durch**
Messen der Luft, die auf natürliche Weise durch das Luftkanalelement (3) strömt, welches vertikal (V) ausgerichtet ist.

## Revendications

1. Dispositif de détection (1) pour déterminer la qualité de l'air intérieur (QAI), dans lequel le dispositif de détection (1) comprend:
un corps (2) ;
au moins une unité de détection (9) pour déterminer les propriétés de l'air ambiant au moyen d'au moins un capteur d'air (13) ; et
au moins une unité d'indication pour indiquer la qualité de l'air détectée ;
et dans lequel le corps (2) comprend au moins un élément de conduit d'air allongé (3) dont les extrémités sont ouvertes, permettant ainsi un écoulement d'air (F) à travers l'élément de conduit d'air (3) ;
la au moins une unité de détection (9) est montée à l'intérieur de l'élément de conduit d'air mentionné (3); et
ledit au moins un dispositif indicateur mentionné comprend au moins une source de lumière (19) pour fournir une indication visuelle de la qualité de l'air détectée ;
**caractérisé en ce que**
ladite au moins une source de lumière (19) est configurée pour éclairer au moins une surface extérieure de l'élément de conduit d'air (3) en réponse à la qualité de l'air intérieur détectée, dans lequel l'élément de conduit d'air (3) est transparent et dans lequel la au moins une source de lumière (19) et l'élément de conduit d'air transparent (3) sont configurés pour coopérer et pour servir de paire d'éclairage fonctionnelle.

2. Dispositif de détection selon la revendication 1, **caractérisé en ce que** l'élément de conduit d'air (3) est un élément tubulaire.

3. Dispositif de détection selon la revendication 1 ou 2, **caractérisé en ce que** l'élément de conduit d'air (3) est au moins partiellement transparent.

4. Dispositif de détection selon l'une quelconque des revendications précédentes 1 à 3, **caractérisé en ce que** le dispositif de détection (1) comprend plusieurs sources de lumière (19) chacune d'elles se situant au moins en partie à l'intérieur de la structure de l'élément de conduit d'air (3) et configurés pour diriger leur lumière directement à l'intérieur du matériau de l'élément de conduit d'air (3).

5. Dispositif de détection selon l'une quelconque des revendications précédentes 1 à 4, **caractérisé en ce que** la couleur de la lumière produite des sources de lumière (19) est configurée pour changer en réponse à la qualité de l'air détectée.

6. Dispositif de détection selon l'une quelconque des revendications précédentes 1 à 5, **caractérisé en ce que**
les sources de lumière mentionnées (19) sont des ampoules à DEL ; et
la couleur de la lumière produite des ampoules à DEL est configurée pour changer en réponse à la qualité de l'air détectée.

7. Dispositif de détection selon l'une quelconque des revendications précédentes 1 à 6, **caractérisé en ce que** le corps (2) comprend au moins un élément de montage (5) pour monter le dispositif de détection (1) de manière amovible.

8. Dispositif de détection selon l'une quelconque des revendications précédentes 1 à 7, **caractérisé en ce que** l'unité de détection (9) est supportée contre les surfaces internes de l'élément de conduit d'air (3).

9. Dispositif de détection selon l'une quelconque des revendications précédentes 1 à 8, **caractérisé en ce que** le dispositif de détection (1) a une direction de montage verticale (V) dans lequel l'élément de conduit d'air (3) est orienté verticalement (V) et l'air circule verticalement à travers le conduit d'air.

10. Dispositif de détection selon l'une quelconque des revendications précédentes 1 à 9, **caractérisé en ce que** l'élément de conduit d'air (3) comprend un tube interne opaque (3b) et un tube externe transparent (3a).

11. Dispositif de détection selon l'une quelconque des revendications précédentes 1 à 10, **caractérisé en ce que**
l'unité de détection (19) comprend plusieurs capteurs d'air ; et
les capteurs d'air (13) sont configurés pour déterminer au moins la température, l'humidité, les concentrations de CO et les concentrations de particules fines.

12. Dispositif de détection selon l'une quelconque des revendications précédentes 1 à 11, **caractérisé en ce que**
l'unité de détection (9) est reliée à au moins une unité de commande (CU, 15) qui comprend au moins un processeur pour traiter les données recueillies par le au moins un capteur d'air (13) et étant transmis à l'unité de commande (CU, 15) ; et
l'unité de commande (CU, 15) est configurée pour déterminer les propriétés de qualité de l'air et pour commander le fonctionnement des sources de lumière (19) en réponse aux propriétés détectées de l'air.

13. Procédé de surveillance de la qualité de l'air intérieur au moyen d'un dispositif de détection portable (1), le procédé comprenant les étapes consistant à :
mesurer les propriétés de l'air ambiant au moyen d'au moins une unité de détection (19) comprenant au moins un capteur d'air (13);
déterminer la qualité de l'air en réponse aux données de mesure recueillies ; et
indiquer visuellement la qualité de l'air déterminée ;
mesurer la qualité de l'air à l'intérieur d'un élément de conduit d'air allongé (3) qui enveloppe ladite au moins une unité de détection (19) ; et
éclairer au moins partiellement l'élément de conduit d'air (3) avec des couleurs différentes en réponse à la qualité de l'air déterminée ;
**caractérisé par**
l'illumination d'au moins une surface externe de l'élément de conduit d'air (3) en réponse à la qualité de l'air intérieur détectée au moyen de la au moins une source de lumière (19) mentionnée, la au moins une source de lumière (19) et le des éléments de conduit d'air transparents (3) coopèrent et servant corne paire d'éclairage fonctionnelle.

14. Procédé selon la revendication 13 ;
**caractérisé par** la mesure de l'air s'écoulant naturellement à travers l'élément de conduit d'air (3) qui est orienté verticalement (V).
